# EUROPEAN PATENT SPECIFICATION

(11) **EP 0 665 434 B1**
(45) Date of publication and mention of the grant of the patent: **08.11.2000**
(21) Application number: 95200151.9
(22) Date of filing: 23.01.1995
(51) Int. Cl.: G01N 33/04, A01J 5/017

(54) **A construction for milking animals**
Vorrichtung zum Melken von Tieren
Dispositif pour traire les animaux

(30) Priority: 28.01.1994 NL 9400131
(43) Date of publication of application: 02.08.1995
(73) Proprietor: MAASLAND N.V., 3155 PD Maasland (NL)
(72) Inventor: van der Lely, Cornelis, CH-6300 Zug (CH)
(74) Representative: Corten, Maurice Jean F.M.

(56) References cited:
- EP-A- 0 091 892
- EP-A- 0 534 565
- EP-A- 0 628 244
- FR-A- 2 691 259
- NL-A- 7 206 038
- NL-A- 8 602 505
- US-A- 3 566 841
- US-A- 3 841 756
- US-A- 4 922 855
- LANDBOUW MECHANISATIE, vol. 44, no.9, September 1993 WAGENINGEN NL, pages 10-11, XP 000394221 'TECHNIEK VAN HET MELKROBOTSYSTEEM'
- LANDTECHNIK, vol. 41, no.5, May 1986 LEHRTE, HANNOVER DE, pages 220-223, H. SCHÖN 'AUTOMATISIERTE MILCHVIEHHALTUNG'
- DATABASE WPI, Week 8821, Derwent Publications Ltd., London, GB, Class , AN 88-145460 & NL 8602505 A

## Description

The invention relates to a construction according to the preamble of claim 1.

US-A-3 841 756 relates to a monitoring unit for monitoring the leucocyte content, the butter fat content and the weight of the milk of the individual cow. This apparatus has the disadvantage that the information about the milk quality is not made directly available, but is first transmitted to a single master unit for processing and printing.

EP-A-0 091 892 describes a robotic milking machine. This machine has the disadvantage that it milks the animals irrespective of the quality of the milk of the individual animal.

The milk obtained by means of the said construction is usually checked to determine its quality and its grades. Milk is a very valuable nutrient. Not only the vitamins, but especially the fat and the albumen are important for health. For this reason not only the milk yield of an animal is recorded, but also the milk is checked for its grade of fat and albumen. On the basis of the quality of the milk it is determined whether the milk is suitable for consumption, whilst payment of the milk may also be effected on this quality basis. As regards controlling of the milk, more particularly as regards checking of e.g. the fat grade and/or the albumen grade, laboratories must be called upon, whereby such laboratories may be located at a large distance from the location where the milk is obtained. This is a time-consuming and expensive matter, which does not improve the quality of the milk.

The invention has for its object to provide a construction of the above-defined type, in which the said disadvantages do not occur or in which these disadvantages are at least obviated to a significant extent.

According to the invention, this is achieved with a construction according to the characterizing part of claim 1. A checking member ca be a member for checking the percentage of the milk fat and/or albumen in the milk. Thus, an operator can immediately determine, for example, the value of the albumen grade of the milk obtained at that instant. When the albumen grade strongly deviates from the anticipated albumen grade of that animal, the operator can decide that, for example, the food supply to the animal must be adapted in order to obtain the desired albumen grade.

The use of a milking robot with automatic sampling gives the farmer time to check the performances of his animals. The milking robot determines whether a given animal is to be milked. According to the invention, the teat cups are connected with the aid of the milking robot to the teats of the relevant animal, whereafter milking can be started. During the milking procedure, in accordance with a feature of the invention, the grade of fat and/or albumen can directly be read from a display screen which is part of the milk checking member.

In an implementation in accordance with the invention, the display screen of the milk checking member may be in a position at a distance from the construction, e.g. in a house or in a farm building. So as to determine the quantity of milk supplied by each animal, the construction includes a milk quantity meter. Than, the fat grade and/or the albumen grade and the quantity of milk obtained during a milking turn can be read from the display screen. In accordance with a still further feature of the invention, the data collected during a sampling of the milk are automatically processed in a computer which is part of the construction. With the aid of the computer it is possible to store, combine and/or to interrelate the above data, so that certain conclusions can be drawn therefrom. Thus, in accordance with a further feature of the invention, the data collected during sampling of the milk can, for example, be transmitted via a modem forming part of the construction to the Herd Registry Office and/or to the Milk Sanitation Office.

In accordance with a feature of the invention, the milk checking member includes a lockable control box. Thus, the milk checking member is only accessible to a limited number of persons. In accordance with a further feature of the invention, the milk checking member is provided with gauging means for gauging the milk checking member. In accordance with a still further feature of the invention, the gauging means include an inlet and an outlet, via which a liquid suitable for gauging, e.g. milk of a known grade, can be fed to and discharged from the milk checking member. In accordance with a feature of the invention, the gauging means further include a reset circuit which can be activated when on the display screen there appears a value which differs from the actual, pre-known value of the grade of said grade liquid. The reset switch has a plurality of correction buttons which are accessible after the said seal has been broken. Since the gauging means can be used at any moment, it is possible to always perform a very accurate determination of a grade by means of the milk checking member.

There is known a variety of apparatuses and systems for measuring the fat and albumen grades, such as a butyrometer. To determine the fat grade, the Gerber, Babcock and Rose-Gottlieb systems are generally known. Measuring albumen-protein can be effected on a more chemical basis in accordance with Kjeldahl. Furthermore, the measurements can be performed by means of sensors.

According to the invention, the milk checking member can employ the Gerber, the Babcock or the Röse-Gottlieb systems to determine the fat grade. In accordance with a further feature of the invention, the milk checking member can employ the Kjeldahl system to determine the albumen grade.

It will be obvious that the invention is not limited to the above systems, but that any other system suitable for determining the grade of fat and/or albumen can be used.

In order to realize a high degree of accuracy and reliability of the conclusions drawn on the basis of the samples, the milking robot is permanently in operation and approximately 1000 sampling operations per animal are performed annually for each milk component. For the purpose of storing the data collected therefrom and, optionally, reading these data of each animal at a later instant, the construction includes, in accordance with a feature of the invention, a cow identification system.

For a better understanding of the invention and to show how the same may be carried into effect, reference will now be made, by way of example, to the accompanying drawings, in which:
Figure 1 is a side view of a construction for milking animals, which construction includes a milk checking member for determining the fat and/or albumen grade, and
Figure 2 shows the milk checking member as shown in Figure 1.

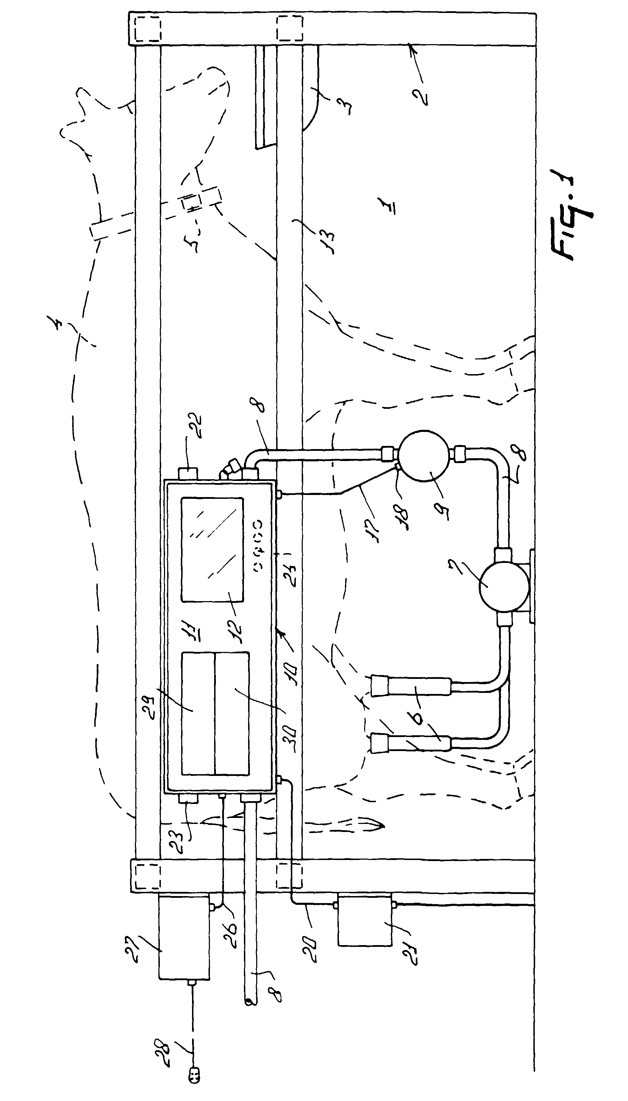

Figure 1 shows a construction for milking animals, which construction comprises a milking box 1 having a railing 2, to which a feed trough 3 is attached. The contours of a cow 4 are shown in the milking box 1. A transponder 5, which is part of a (non-shown) cow identification system, is disposed around the neck of the cow 4. By means of the cow identification system, by reading the transponder 5, the identity of the animal 4 present in the milking box 1 can be determined.

The construction for milking animals comprises a (non-shown) milking robot, with the aid of which teat cups 6 can automatically be connected to the teats of an animal 4 and the milking process can be performed.

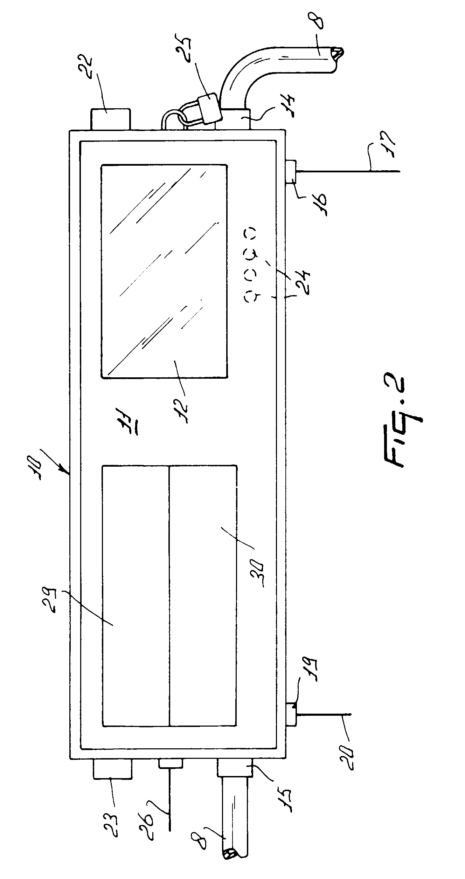

As is shown in Figure 1, the construction for milking animals includes a pump 7, with the aid of which the milk obtained can be pumped through a milk line 8 to a (non-shown) milk tank. In the milk line 8 there is incorporated a milk quantity meter 9, which determines the quantity of milk supplied by an animal during each milking turn. The milk quantity meter 9 may be in the form of a flow meter, a milk collecting glass, a weight measuring device, etc. In addition, the milk line 8 further includes a milk checking member 10 for the determination of fat and/or albumen grade. The milk checking member 10 comprises a control box 11 with a display screen 12. The control box 11 is disposed aside of the milking box 1 on a cross-beam 13 of the railing 2. As is shown in Figure 2, at one side of the control box 11 there is arranged a first connecting nipple 14, to which the milk supply line 8 is connected. At the other side of the control box 11 there is arranged a second connecting nipple 15, to which the milk discharge line 8 is connected, through which line the milk, after having flown through the milk checking member, is discharged to the milk tank. A first terminal for the electric line 17 of a sensor 18 of the milk quantity meter 9 is arranged at the bottom side of the control box 11. Via the electric line 17, the sensor 18 applies to the milk checking member 10 a signal, which represents the rate of flow in the milk line 8.

At the bottom side of the control box 11 there is a further, second terminal 19, to which can be connected an electric line 20 which provides the milk checking member 10 with current. As is shown in Figure 1, an emergency energy source 21 is incorporated in the electric line 20, which is switched-on in the event of a power cut.

The milk checking member 10 further includes gauging means, with the aid of which the grade determined by the milk checking member 10 can be gauged and displayed on the display screen 12. The gauging means have a third connecting nipple 22, via which the types of milk or other substances composed specifically for gauging purposes can be supplied to the milk checking member 10. The substances supplied for gauging via the third connecting nipple 22 are automatically discharged through a connecting nipple 23, which is disposed on the control box 11 and to which a (non-shown) reservoir can be connected. In addition, the gauging means have a plurality of correction buttons 24, which are accommodated in the control box 11 and with the aid of which the digits displayed on the display screen 12 can be adjusted, when there is displayed a grade different from the previously known grade of the liquid suitable for gauging. So as to prevent an unauthorized person from getting access to the interior of the control box 11, this control box is provided with a seal 25, which must be broken to allow access thereto. For example, an inspector can perform one or several times per annum a gauging of the milk checking member, using three types of milk having known fat, albumen, etc. grade (e.g. 4%, 5% and 6%). In the case of deviating values, the inspector can adjust the milk checking member by opening the sealed wall and setting the correct values by means of the correction buttons 24.

In addition, a modem 27 is connected to the control box 11 via an electric line 26. By means of the modem 27, data collected by the milk checking member 10 can be transmitted via a telephone line 28 to a remote computer and/or display screen. The milk checking member may also be connected to the Herd Registry Office and/or to the Milk Sanitation Office. A person working at the Herd Registry Office can look into the data collected for a given animal and consequently determine the quality of her milk, without paying a visit to the farmer.

The computer installed in the house or in the farm building is preferably provided with a management program which can combine the data collected by the milk checking member 10, so that thereafter the farmer can read useful information on e.g. the health of the animal, the quality of the milk, how the lactation period proceeds, etc.

Since at the end of the lactation period the albumen grade, the quantity of milk supplied in each milking turn and the fat grade may vary significantly, the data of approximately one thousand samples of the aforesaid components per animal are stored in the said computer, so that on the basis of these data the reliability of the conclusions related thereto is increased.

The arrangement described in the foregoing operates as follows:

When an animal enters the milking box 1, the identity of the animal is determined by means of the cow identification system. Based on the period of time elapsed since the last time the relevant animal was milked, it is determined whether the animal is to be milked. Since the construction includes a milking robot, the teat cups are connected automatically to the teats of the animal to be milked. The pump 7 is thereafter activated and the milk obtained is then pumped to the milk tank through the milk line 8. The milk then first passes the milk quantity meter 9 with the sensor 18, which applies a signal to the milk checking member 10 via the electric line 17. The display screen 12 of the milk checking member then permanently displays the quantity of milk supplied during the milking turn. Of the milk supplied via the milk line 8 to the milk checking member, the grade of a plurality of components is analyzed in the analyzing members 29, 30 which are accommodated in the control box and which make use of known per se techniques, such as the Gerber, the Babcock and the Röse-Gottlieb systems for determining the fat grade and the Kjeldahl system for determining albumen and protein. The determination of the grade of a milk component is also possible by means of sensors. During and/or after the milking turn, the farmer can read the fat grade, the albumen grade and the milk yield from the display screen 12. These data are optionally also transferred via the modem 27 to the (management) computer, where they are stored and processed. On the basis of aforesaid data, the farmer can accurately check the performances of his animals and, if necessary, can make changes in e.g. the feeding pattern of the animal and in the composition of the fodder.

## Claims

1. A construction including at least one implement for milking animals, such as cows, said implement being provided with a milk quantity meter (9) and a milk checking member (10) for the determination of the grade of fat and/or albumen in the milk, obtained from the individual animals during milking, characterized in that the implement is constituted by a milking robot for automatically milking animals, and in that the checking member (10) is connected to the quantity meter (9) and the checking member (10) is provided with a display screen (12) on which the quantity of milk obtained from an individual animal during milking and the fat grade and/or the albumen grade thereof can be read.

2. A construction as claimed in claim 1, characterized in that the data collected during milking in a milk sampling operation are automatically processed in a computer which is part of the construction.

3. A construction as claimed in claim 2, characterized in that the data collected during the milk sampling operation are transmitted via a modem (27) forming part of the construction to the Herd Registry Office and/or to the Milk Sanitation Office.

4. A construction as claimed in one or more of the preceding claims, characterized in that the milk checking member (10) includes a lockable control box.

5. A construction as claimed in any one of the preceding claims, characterized in that the milk checking member (10) is provided with gauging means.

6. A construction as claimed in claim 5, characterized in that the gauging means include an inlet and an outlet (22, 23), via which a liquid suitable for gauging, such as milk of a known grade, can be fed to and discharged from the milk checking member (10).

7. A construction as claimed in claim 6, characterized in that the gauging means include a reset circuit which can be activated when the display screen (12) displays a value which differs from the actual, pre-known value of the grade of said gauging liquid.

8. A construction as claimed in any one of the preceding claims, characterized in that the milk checking member (10) employs the fat grade determination in accordance with the Gerber system.

9. A construction as claimed in any one of the claims 1 to 7, characterized in that the milk checking member (10) employs the fat grade determination in accordance with the Babcock system.

10. A construction as claimed in any one of the claims 1 to 7, characterized in that the milk checking member (10) employs the fat grade determination in accordance with the Rose-Gottlieb system.

11. A construction as claimed in any one of the claims 1 to 7, characterized in that the milk checking member (10) employs the albumen grade determination in accordance with the Kjeldahl system.

12. A construction as claimed in any one of the preceding claims, characterized in that the construction includes a cow identification system.

## Patentansprüche

1. Anlage mit mindestens einer Vorrichtung zum Melken von Tieren, wie z. B. Kühen, wobei die Vorrichtung einen Milchmengenmesser (9) und eine Milchprüfvorrichtung (10) zur Ermittlung des Gehaltes an Fett und/oder Eiweiß der von den einzelnen Tieren während des Melkens gewonnenen Milch aufweist,
dadurch gekennzeichnet, daß die Vorrichtung durch einen Melkroboter zum automatischen Melken von Tieren gebildet ist, und daß die Prüfvorrichtung (10) mit dem Milchmengenmesser (9) verbunden ist und einen Bildschirm (12) aufweist, von dem die von einem einzelnen Tier während des Melkens gewonnene Milchmenge sowie der Fettgehalt und/oder der Eiweißgehalt ablesbar sind.

2. Anlage nach Anspruch 1,
dadurch gekennzeichnet, daß die Daten, die während des Melkens bei einer Milchprobenentnahme gesammelt werden, automatisch in einem Computer verarbeitet werden, der Teil der Anlage ist.

3. Anlage nach Anspruch 2,
dadurch gekennzeichnet, daß die während der Milchprobenentnahme gesammelten Daten über ein Modem (27), das Teil der Anlage ist, an das Herdenregisteramt und/oder das Milchhygieneamt übermittelt werden.

4. Anlage nach einem oder mehreren der vorhergehenden Ansprüche,
dadurch gekennzeichnet, daß die Milchprüfvorrichtung (10) einen verschließbaren Schaltschrank aufweist.

5. Anlage nach einem der vorhergehenden Ansprüche,
dadurch gekennzeichnet, daß die Milchprüfvorrichtung (10) eine Eichvorrichtung aufweist.

6. Anlage nach Anspruch 5,
dadurch gekennzeichnet, daß die Eichvorrichtung einen Einlaß und einen Auslaß (22, 23) aufweist, über welche eine zum Eichen geeignete Flüssigkeit, wie z. B. Milch mit einem bekannten Gehalt, in die Milchprüfvorrichtung (10) eingeleitet und aus ihr abgeleitet werden kann.

7. Anlage nach Anspruch 6,
dadurch gekennzeichnet, daß die Eichvorrichtung einen Löschkreis enthält, der aktiviert werden kann, wenn der Bildschirm (12) einen Wert anzeigt, der von dem tatsächlichen vorbekannten Wert des Gehaltes der Eichflüssigkeit abweicht.

8. Anlage nach einem der vorhergehenden Ansprüche,
dadurch gekennzeichnet, daß die Milchprüfvorrichtung (10) die Bestimmung des Fettgehaltes nach dem Gerber-System vornimmt.

9. Anlage nach einem der Ansprüche 1 bis 7,
dadurch gekennzeichnet, daß die Milchprüfvorrichtung (10) die Bestimmung des Fettgehaltes nach dem Babcock-System vornimmt.

10. Anlage nach einem der Ansprüche 1 bis 7,
dadurch gekennzeichnet, daß die Milchprüfvorrichtung (10) die Bestimmung des Fettgehaltes nach dem Röse-Gottlieb-System vornimmt.

11. Anlage nach einem der Ansprüche 1 bis 7,
dadurch gekennzeichnet, daß die Milchprüfvorrichtung (10) die Bestimmung des Eiweißgehaltes nach dem Kjeldahl-System vornimmt.

12. Anlage nach einem der vorhergehenden Ansprüche,
dadurch gekennzeichnet, daß die Anlage ein Kuhidentifikationssystem aufweist.

## Revendications

1. Installation comportant au moins un dispositif pour la traite d'animaux, tels que des vaches, ledit dispositif étant muni d'un compteur de quantité de lait (9) et d'un organe de vérification du lait (10) pour déterminer le niveau de matière grasse et/ou d'albumine dans le lait, obtenu à partir d'animaux individuels lors de la traite, caractérisée en ce que le dispositif est constituée d'un robot de traite pour la traite automatique d'animaux, et en ce que l'organe de vérification (10) est connecté au compteur de quantité (9), et en ce que l'organe de vérification (10) est muni d'un dispositif d'affichage (12) sur lequel la quantité de lait obtenue à partir d'un animal individuel lors de la traite et le niveau de matière grasse et/ou d'albumine de celui-ci peuvent être lus.

2. Installation selon la revendication 1, caractérisée en ce que les données recueillies lors de la traite par un échantillonnage du lait sont automatiquement traitées dans un ordinateur qui est une partie de l'installation.

3. Installation selon la revendication 2, caractérisée en ce que les données recueillies lors de l'échantillonnage du lait sont transmises via un modem (27) formant une partie de la construction à l'Office d'Enregistrement des Troupeaux et/ou à l'Office Sanitaire du Lait.

4. Installation selon l'une quelconque des revendications précédentes, caractérisée en ce que l'organe de vérification du lait (10) comporte un compartiment de commande pouvant être verrouillé.

5. Installation selon l'une quelconque des revendications précédentes, caractérisée en ce que l'organe de vérification du lait (10) est muni de moyens de calibrage.

6. Installation selon la revendication 5, caractérisée en ce que les moyens de calibrage comportent une entrée et une sortie (22, 23), via lesquelles un liquide approprié pour le calibrage, tel que du lait d'un niveau connu, peut être alimenté vers l'organe de vérification du lait (10) et évacué de celui-ci.

7. Installation selon la revendication 6, caractérisée en ce que les moyens de calibrage comportent un circuit de remise à zéro qui peut être actionné lorsque le dispositif d'affichage (12) affiche une valeur qui est différente de la valeur réelle précédemment connue du niveau dudit liquide de calibrage.

8. Installation selon l'une quelconque des revendications précédentes, caractérisée en ce que l'organe de vérification du lait (10) utilise la détermination du niveau de matière grasse selon le système Gerber.

9. Installation selon l'une quelconque des revendications 1 à 7, caractérisée en ce que l'organe de vérification du lait (10) utilise la détermination du niveau de matière grasse selon le système Babcock.

10. Installation selon l'une quelconque des revendications 1 à 7, caractérisée en ce que l'organe de vérification du lait (10) utilise la détermination du niveau de matière grasse selon le système Rôse-Gottlieb.

11. Installation selon l'une quelconque des revendications 1 à 7, caractérisée en ce que l'organe de vérification du lait (10) utilise la détermination du niveau d'albumine selon le système Kjeldahl.

12. Installation selon l'une quelconque des revendications précédentes, caractérisée en ce que l'agencement comporte un système d'identification de vache.
